# EUROPEAN PATENT SPECIFICATION

(11) **EP 3 373 014 B1**
(45) Date of publication and mention of the grant of the patent: **23.12.2020**
(21) Application number: 16857448.1
(22) Date of filing: 19.10.2016
(51) Int. Cl.: G01N 33/92

(54) **DIAGNOSTIC MARKER FOR CORONARY ARTERY DISEASE**
DIAGNOSTISCHER MARKER FÜR KORONARARTERIENERKRANKUNG
MARQUEUR POUR LE DIAGNOSTIC DE LA CORONAROPATHIE

(30) Priority: 20.10.2015 JP 2015206004
(43) Date of publication of application: 12.09.2018
(73) Proprietor: MedImmune, LLC, Gaithersburg, MD 20878 (US)
(72) Inventor: ONO, Takashi, Toyonaka-shi Osaka 561-0825 (JP); HAMADA, Tadateru, Toyonaka-shi Osaka 561-0825 (JP); SHIGAKI, Shuhei, Toyonaka-shi Osaka 561-0825 (JP); MORITA, Atsushi, Toyonaka-shi Osaka 561-0825 (JP)
(74) Representative: AstraZeneca
(86) International application number: PCT/JP2016/080901
(87) International publication number: WO 2017/069135

(56) References cited:
- WO-A1-2011/063470
- WO-A1-2011/063470
- WO-A1-2015/163342
- WO-A1-2015/163342
- "PUBCHEM substance/57389388, 1-(9Z,12Z-octadecadienoyl)-2-(9Z,12Z,15Z-o ctadecatrienoyl)-sn-glycero-3-phospho-(1'- myo-inositol),", National Center for Biotechnology Information. PubChem Substance Database; SID=57389388, https://pubchem.ncbi.nlm.nih.gov/substance /57389388 (accessed Feb. 26, 2019). , 31 March 2009 (2009-03-31), XP002789237, Retrieved from the Internet: URL:https://pubchem.ncbi.nlm.nih.gov/subst ance/57389388/version/1#section=Top [retrieved on 2019-02-26]
- HICKS A M ET AL: "Unique molecular signatures of glycerophospholipid species in different rat tissues analyzed by tandem mass spectrometry", BIOCHIMICA ET BIOPHYSICA ACTA (BBA) - MOLECULAR AND CELL BIOLOGY OF LIPIDS, ELSEVIER, AMSTERDAM, NL, vol. 1761, no. 9, 2 June 2006 (2006-06-02) , - 1 September 2006 (2006-09-01), pages 1022-1029, XP028040095, ISSN: 1388-1981, DOI: 10.1016/J.BBALIP.2006.05.010 [retrieved on 2006-09-01]
- M. KURANO ET AL: "Possible Involvement of Minor Lysophospholipids in the Increase in Plasma Lysophosphatidic Acid in Acute Coronary Syndrome", ARTERIOSCLEROSIS, THROMBOSIS, AND VASCULAR BIOLOGY., vol. 35, no. 2, 1 January 2014 (2014-01-01), - 25 November 2014 (2014-11-25), pages 463-470, XP055377407, US ISSN: 1079-5642, DOI: 10.1161/ATVBAHA.114.304748
- HIROAKI TAKEDA ET AL: "Lipidomic analysis of plasma lipoprotein fractions in myocardial infarction-prone rabbits", JOURNAL OF BIOSCIENCE AND BIOENGINEERING, vol. 120, no. 4, 8 July 2015 (2015-07-08), - 1 October 2015 (2015-10-01), pages 476-482, XP055377410, NL ISSN: 1389-1723, DOI: 10.1016/j.jbiosc.2015.02.015
- KIM JUNHWAN ET AL: "Phospholipid alterations in the brain and heart in a rat model of asphyxia-induced cardiac arrest and cardiopulmonary bypass resuscitation", MOLECULAR AND CELLULAR BIOCHEMISTRY, NORWELL, MA, US, vol. 408, no. 1, 10 July 2015 (2015-07-10) , pages 273-281, XP035547488, ISSN: 0300-8177, DOI: 10.1007/S11010-015-2505-0 [retrieved on 2015-07-10]
- MICHIYO OKUDAIRA ET AL: "Separation and quantification of 2-acyl-1-lysophospholipids and 1-acyl-2-lysophospholipids in biological samples by LC-MS/MS", JOURNAL OF LIPID RESEARCH, vol. 55, no. 10, 29 October 2014 (2014-10-29), pages 2178-2192, XP055456959, US ISSN: 0022-2275, DOI: 10.1194/jlr.D048439
- KOISTINEN KAISA M ET AL: "Quantitative lysophospholipidomics in human plasma and skin by LC-MS/MS", ANALYTICAL AND BIOANALYTICAL CHEMISTRY, SPRINGER, DE, vol. 407, no. 17, 25 January 2015 (2015-01-25), pages 5091-5099, XP035487896, ISSN: 1618-2642, DOI: 10.1007/S00216-014-8453-9 [retrieved on 2015-01-25]
- MAKOTO KURANO ET AL: "Different origins of lysophospholipid mediators between coronary and peripheral arteries in acute coronary syndrome", JOURNAL OF LIPID RESEARCH, vol. 58, no. 2, 22 December 2016 (2016-12-22), pages 433-442, XP055600150, US ISSN: 0022-2275, DOI: 10.1194/jlr.P071803 -& MAKOTO KURANO ET AL: "Supplemental Data: Different origins of lysophospholipid mediators between coronary and peripheral arteries in acute coronary syndrome", JOURNAL OF LIPID RESEARCH, vol. 58, no. 2, 22 December 2016 (2016-12-22), pages 433-442, XP055600154, US ISSN: 0022-2275, DOI: 10.1194/jlr.P071803
- MAKOTO KURANO: "Kandomyaku Shikkan Kanja ni Okeru Serotonin, Kokando CRP to Lysophospholipid no Sokan", Rinsho Kagaku, vol. 43, no. 1, 2014, page 238, XP009509732,
- KURANO MAKOTO: "Possible involvement of minor lysophospholipids in the increase in plasma lysophosphatidic acid in acute coronary syndrome", Arteriosclerosis, thrombosis, and vascular biology, vol. 35, no. 2 February 2015 (2015-02), pages 463-70, XP055377407, [retrieved on 2014-11-25]
- TAKEDA HIROAKI: "Lipidomic analysis of plasma lipoprotein fractions in myocardial infarction-prone rabbits", Journal of Bioscience and Bioengineering, vol. 120, no. 4 October 2015 (2015-10), pages 476-482, XP055377410, [retrieved on 2015-07-08]
- MAZEREEUW GRAHAM: "Omega-3/omega-6 fatty acid ratios in different phospholipidic classes and depressive symptoms in coronary artery disease patients", Brain, Behavior, and immunity, vol. 53 March 2016 (2016-03), pages 54-58, XP029452076, [retrieved on 2015-12-14]
- KURANO MAKOTO: "Possible Involvement of Minor Lysophospholipids in the Pathogenesis of Acute Coronary Syndrome", The Journal of Japanese Society on Thrombosis and Hemostasis, vol. 27, no. 2 , page 139, XP009511441, ISSN: 1880-8808

## Description

### Technical Field

The present invention relates to the diagnosis of coronary artery disease. In detail, the present invention relates to the diagnosis of coronary artery disease using phosphatidylinositol (36:5) as a molecular marker.

### Prior Art

Heart disease is second to cancer as a cause of death among the Japanese (statistical data of Statistics Bureau, Ministry of Internal Affairs and Communications), and the majority of this is coronary artery disease (ischemic heart disease). Coronary artery disease is disease that is a result of poor blood flow to the coronary arteries, which supply blood to the myocardium, and insufficient blood supply to the myocardium. Arteriosclerosis is a primary cause of coronary artery disease. A reduction in the amount of blood supplied and interruption of blood flow to the myocardium occur due to constriction on the inside of the blood vessels. The amount of oxygen supplied from the blood does not satisfy the demand for oxygen required by the myocardium and a state of oxygen deficiency is produced. This leads to angina and myocardial infarction.

The leading cause of arteriosclerosis is lipid metabolism anomalies, including an increase in LDL cholesterol. Consequently, lipids that change with arteriosclerosis are potential candidates for coronary artery disease biochemical markers. In particular, LDL cholesterol is a marker and causative factor from arteriosclerosis to coronary artery disease. However, sufficient lowering of LDL cholesterol by statin administration does not always prevent a cardiovascular event (Non-Patent Document 2). It cannot be said that LDL cholesterol is a sufficient coronary artery disease marker or therapeutic target.

Regarding markers of coronary artery disease, although a patent application (Patent Document 1) exists for the diagnosis of coronary artery disease using the amount of at least two lipid analytes, such as phosphatidylinositol (36:1), in the blood as the indicator, a marker with which coronary artery disease can be diagnosed by a more convenient assessment method is not known.

### Prior Art Documents

### Patent Documents

Patent Document 1: International Early Disclosure No. 2011/063470 relates generally to the field of diagnostic and prognostic assays for heart disease.

### Non-Patent Documents

Non-Patent Document 1: American Journal of Cardiology, 2008, volume 101, No. 8, Supplement, pages S27-S35; Non-patent Document 2: Biochimica et Biophysica Acta, 2006, volume 1761, pages 1022-1029 refers to unique molecular signatures of glycerophospholipid species in different rat tissues analyzed by tandem mass spectrometry; Non-patent Document 3: Arteriosclerosis, Thrombosis, and Vascular Biology, 2015, volume 35, pages 463-470 refers to the possible involvement of minor lysophospholipids in the increase in plasma lysophosphatidic acid in acute coronary syndrome; Non-patent Document 4: Journal of Bioscience and Bioengineering, 2015, volume 120, pages 476-482 refers to the lipidomic analysis of plasma lipoprotein fractions in myocardial infarction-prone rabbits; Non-patent Document 5:Mol Cell Biochem, 2015, volume 408, pages 273-281 refers to Phospholipid alterations in the brain and heart in a rat model of asphyxia-induced cardiac arrest and cardiopulmonary bypass resuscitation; Non-patent Document 6: https://pubchem.ncbi.nlm.nih.gov/substance/57389388, relates to 1-18:2-2-18:3-phosphatidylinositol.

### Summary of Invention

### Problem to Be Solved By Invention

The present invention addresses the problem of developing a convenient method for diagnosing arteriosclerosis.

### Means for Solving Problem

The inventors conducted in-depth research in the light of the above-mentioned problem and as a result, successfully perfected the present invention upon discovering that coronary artery disease can be diagnosed by using phosphatidylinositol (36:5) in peripheral blood as a marker.

That is, the present invention relates to the following:
**(1)** An *in vitro* method for detecting coronary artery disease, comprising a step for determining the amount of phosphatidylinositol (36:5) in a blood sample derived from a subject, wherein the fact that the determined value is smaller than a reference value corresponding to the amount of phosphatidylinositol (36:5) in a control sample derived from a person without coronary artery disease, is used as an indicator that the test sample is a sample derived from a person with coronary artery disease.
**(2)** Use of phosphatidylinositol (36:5) as a marker for the *in vitro* detection of coronary artery disease.

### Effect of Invention

According to the present invention, coronary artery disease can be conveniently diagnosed because, for instance, phosphatidylinositol (36:5) can be used alone as a marker. The present invention is further characterized in that the stress on a subject is reduced because determination using blood or serum is possible.

### Brief Description of Drawings

**[****Figure 1]** Figure 1 is a drawing showing an example of phosphatidylinositol (36:5).
**[****Figure 2]** Figure 2 is a drawing showing an example of lysophosphatidylinositol (20:5).
**[****Figure 3]** Figure 3 shows the relative values of the phosphatidylinositol (36:5) concentrations in peripheral blood of the control group and the coronary artery disease group.
**[****Figure 4]** Figure 4 shows the ROC curve for phosphatidylinositol (36:5) .
**[****Figure 5]** Figure 5 shows the relative values of the lysophosphatidylinositol (20:5) concentrations in peripheral blood of the control group and the coronary artery disease group.
**[****Figure 6]** Figure 6 shows the ROC curve for lysophosphatidylinositol (20:5).

### Detailed description

The terminology used in the specification is according to the definitions normally used in this field unless otherwise specified.

Specific embodiments of the present invention are given below, but the present invention is not restricted to these embodiments.

Beyond the scope of the invention, which is defined exclusively by the appended claims, the present disclosure provides a more generic framework for the diagnosis of coronary artery disease, using phosphatidylinositol (36:5) or lysophosphatidylinositol (20:5) as a molecular marker.

Thus, the present disclosure provides a method for judging whether a subject has developed coronary artery disease (is a person with coronary artery disease).

This method relates to
a method for detecting coronary artery disease, comprising a step for determining the amount of phosphatidylinositol (36:5) or lysophosphatidylinositol (20:5) in a test sample derived from a subject, wherein the determined value is used as an indicator that the sample is derived from a person with coronary artery disease.

Moreover, the present disclosure
includes a method for detecting coronary artery disease, comprising a step for determining the amount of phosphatidylinositol (36:5) or lysophosphatidylinositol (20:5) in a test sample derived from a subject, wherein here the fact that the amount of phosphatidylinositol (36:5) or lysophosphatidylinositol (20:5) in the test sample is smaller than a reference value is used as an indicator that the test sample is a sample derived from a person with coronary artery disease.

Furthermore, the present disclosure
includes a method for detecting coronary artery disease, comprising a step for determining the amount of phosphatidylinositol (36:5) or lysophosphatidylinositol (20:5) in a test sample derived from a subject, wherein here the fact that the amount of phosphatidylinositol (36:5) or lysophosphatidylinositol (20:5) in the test sample is smaller than the amount in a control sample derived from a person without coronary artery disease is used as an indicator that the test sample is a sample derived from a person with coronary artery disease.

That is, screening (diagnosis) of a person with coronary artery disease is possible by using the above-mentioned method to determine that a sample is derived from a person with coronary artery disease.

The characterizing feature of the present disclosure is the use of the amount of phosphatidylinositol (36:5) or lysophosphatidylinositol (20:5) in a test sample derived from a subject as a molecular marker. The "test sample" collected from a subject includes samples derived from urine, whole blood, plasma, serum, or blood, and in terms of convenience, is preferably a sample that can be prepared from peripheral blood. The peripheral blood can be collected from any site, but is generally collected from a vein of a subject. The present disclosure can be conveniently executed with reduced stress on the subject by collecting peripheral blood from an arm vein. Preferably plasma is separated from the collected peripheral blood and the amount of phosphatidylinositol (36:5) or lysophosphatidylinositol (20:5) in the plasma is determined. Phosphatidylinositol (36:5) or lysophosphatidylinositol (20:5) can be used alone as the coronary artery disease marker or can be used in combination with other markers known to be capable of detecting coronary artery disease.

The phosphatidylinositol to be determined in the present disclosure is an acidic phospholipid having inositol at a polar group and is also known by the abbreviation PI. Various molecular species exist with different combinations of fatty acids at positions 1 and 2. For instance, the notation phosphatidylinositol (38:4) is used for the case where ester-bonded fatty acids at positions 1 and 2 have a total of 38 carbons and a total of 4 double bonds as estimated on the basis of the molecular weight assumed from the results of analysis of the phosphatidylinositol molecular species by mass analysis.

The phosphatidylinositol to be determined in the present disclosure has ester-bonded fatty acids at positions 1 and 2 where the total number of carbons is 36 and the total number of double bonds is 5 as estimated on the basis of the molecular weight assumed from the results of analysis by mass analysis, and is therefore phosphatidylinositol (36:5) (phosphatidylinositol having fatty acid residues at positions sn-1, 2 where the total number of carbons is 36 and the total number of double bonds is 5) .

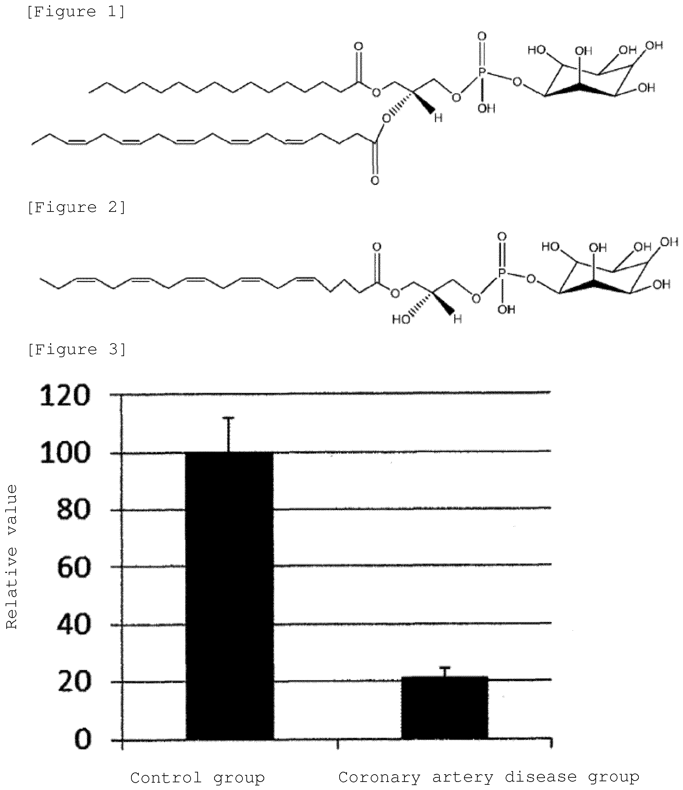

Figure 1 shows an example of phosphatidylinositol (36:5) to be determined in the present disclosure.

The lysophosphatidylinositol to be determined in the present disclosure is an acidic phospholipid having inositol at a polar group and is also known by the abbreviation LPI. Various molecular species exist with different fatty acid contents. For instance, the notation lysophosphatidylinositol (18:1) is used for the case where ester-bonded fatty acids have a total of 18 carbons and a total of 1 double bond as estimated on the basis of the molecular weight assumed from the results of analysis of the lysophosphatidylinositol molecular species by mass analysis.

The lysophosphatidylinositol to be determined in the present disclosure has ester-bonded fatty acids where the total number of carbons is 20 and the total number of double bonds is 5 as estimated on the basis of the molecular weight assumed from the results of analysis by mass analysis, and is therefore lysophosphatidylinositol (20:5) (lysophosphatidylinositol having fatty acid residues where the total number of carbon atoms is 20 and the total number of double bonds is 5).

Figure 2 shows an example of the lysophosphatidylinositol (20:5) to be determined in the present disclosure.

Examples of methods for determining the amount of phosphatidylinositol and lysophosphatidylinositol are mass analysis and immunoassay.

Mass analysis can be performed while referring to the following examples and the Journal of Separation Science, 2012, Volume 35, pages 1845-1853 or Analytical Biochemistry, 2008, Volume 375, pages 124-131, for instance.

Immunoassay can be a conventional immunoassay. Examples of conventional immunoassay are ELISA, RIA, and western blot.

The type, derivation, and the like of the antibody used for immunoassay is not particularly restricted as long as the antibody has the ability to specifically bind with phosphatidylinositol (36:5) or lysophosphatidylinositol (20:5). Monoclonal antibody and polyclonal antibody can be used, but monoclonal antibody is preferred.

The antibody can be prepared by conventional methods using phosphatidylinositol (36:5) or lysophosphatidylinositol (20:5) as the immunogen. Phosphatidylinositol (36:5) and lysophosphatidylinositol (20:5) are low-molecular-weight compounds and when used alone as the immunogen, production of antibody is likely to be difficult. Therefore, preferably a compound with a carrier protein is prepared. In general, the mammal that is inoculated with the immunogen is a rabbit, goat, sheep, mouse, rat, and the like. Immunization can be performed by a conventional method, such as administration of the immunogen to a mammal intravenously, intradermally, subcutaneously, or intraperitoneally. After immunization, the antiserum is collected from the mammal and polyclonal antibody is made, or plasma cells (immune cells) are collected and monoclonal antibody is made by a method such as described in "Molecular and Cellular Biology Basic Experimental Methods" (Nanedo Publishers, Takekazu Horie et al., 1994) .

The antibody can be labeled by a label that can be detected and quantitatively determined. Radioactivity and fluorescence are known labels and can be selected as appropriate. For example, a biotinylated antibody is easily detected.

According to the method for detecting coronary artery disease of the present disclosure, the amount of phosphatidylinositol (36:5) or lysophosphatidylinositol (20:5) in a test sample obtained from a subject is compared with a reference value and when the amount is smaller than the reference value, the test sample is screened as a sample derived from a person with coronary artery disease.

The term "reference value" is defined as the amount of phosphatidylinositol (36:5) or lysophosphatidylinositol (20:5) in a control sample derived from a person without coronary artery disease, for instance. The amount in the control sample is preferably the average value of multiple samples. The amount in the control sample can be determined simultaneously with the test sample, or a cut-off value can be set by predetermination.

The phosphatidylinositol (36:5) or lysophosphatidylinositol (20:5) is much less in a sample derived from a person with coronary artery disease than in a control sample derived from a person without coronary artery disease. Consequently, in the method for detecting coronary artery disease of the present disclosure, when the amount of phosphatidylinositol (36:5) or lysophosphatidylinositol (20:5) in a test sample, or the value calculated on the basis of the amount of phosphatidylinositol (36:5) or lysophosphatidylinositol (20:5), is significantly smaller than the amount in the above-mentioned "control sample", or is smaller than the pre-set cut-off value, it can be said that there is a strong possibility that the test sample is a sample derived from a person with coronary artery disease.

Note that the "cut-off value" in the present disclosure is defined as a value for differentiating between a person with coronary artery disease and a person without coronary artery disease by comparison of the amount of phosphatidylinositol (36:5) or lysophosphatidylinositol (20:5) obtained by the above-mentioned method for detecting coronary artery disease, or the value calculated on the basis of the amount of phosphatidylinositol (36:5) or lysophosphatidylinositol (20:5). Although the method for setting the cut-off value is not particularly restricted, the cut-off value can be set using an ROC curve as in the following examples.

When coronary artery disease is detected using a cut-off value, the cut-off value varies with the method for determining the marker. Consequently, preferably the determined values of a person without coronary artery disease and a person with coronary artery disease are confirmed in advance for a target marker, the cut-off value is set, and detection is performed according to the cut-off value. In accordance with the examples of the present disclosure, when a marker is determined using a subject's serum, coronary artery disease can be detected by referring to the numbers obtained in the examples of the present disclosure.

Note that when 2 or more determined values, such as the value of a test sample and the value of a control sample, are compared, comparison can be by using one or multiple statistical analyses (such as the t-test, Welch's t-test, the Wilcoxon ranked sum test, ANOVA, recursive decomposition, or random forests).

The present disclsoure provides a kit for detecting coronary artery disease comprising phosphatidylinositol (36:5) or lysophosphatidylinositol (20:5). The present disclosure further provides a kit, characterized in containing antibody that specifically recognizes phosphatidylinositol (36:5) or lysophosphatidylinositol (20:5). These kits can be used in the method for detecting coronary artery disease of the present disclosure.

In addition, these kits further comprise one or more structural elements necessary for assay in general. The structural elements can be reference standard, reagent (diluent, buffer, and the like), containers and/or devices.

Furthermore, the present disclosure makes possible the treatment of a person who has been diagnosed with coronary artery disease by the above-mentioned method using phosphatidylinositol (36:5) or lysophosphatidylinositol (20:5). Specifically, the present disclosure is a method for treating coronary artery disease comprising
a step for detecting coronary artery disease wherein in the above-mentioned method for detecting coronary artery disease, the fact that the amount in a test sample is smaller than the amount in a control sample derived from a person without coronary artery disease is used as an indicator that the test sample is derived from a person with coronary artery disease and
a step for treating a subject in whom coronary artery disease has been detected by one or more treatments selected from the group consisting of pharmacotherapy, surgery, exercise therapy and diet therapy.

That is, the present disclosure includes a method for treating a person with coronary artery disease by
(a) determining the phosphatidylinositol (36:5) or lysophosphatidylinositol (20:5) concentration in a test sample derived from a subject,
(b) detecting coronary artery disease using the determined value as an indicator that the sample was derived from a person with coronary artery disease, and
(c) treating the subject in whom coronary artery disease has been detected by one or more treatments selected from the group consisting of pharmacotherapy, surgery, exercise therapy, and diet therapy.

Examples of pharmacotherapy are nitric acid medicines, β blockers, calcium antagonists, antiplatelet drugs (aspirin, and the like) and cholesterol-lowering drugs.

Examples of surgery are coronary artery bypass grafting (CABG), balloon angioplasty, and stent placement.

The present disclosure will now be described in further detail and more specifically using non-limiting examples.

### Example 1

### Diagnosis of coronary artery disease using phosphatidylinositol (36:5) in peripheral blood as molecular marker

In order to investigate the performance of phosphatidylinositol (36:5) as a novel marker in coronary artery disease, the phosphatidylinositol (36:5) concentration in plasma was compared between 20 people in the control group and 20 people in the coronary artery disease group.

The subjects were patients with coronary artery disease, and plasma samples derived from patients that were provided by Proteogenex were used. The control group was adults without coronary artery disease. This experiment was performed after obtaining the approval of the Review Board for Ethical Research using Human Tissue and Genes of Shionogi & Company. The blood was collected after obtaining written consent and stored frozen at -80°C in aliquots.

The phosphatidylinositol (36:5) concentration in peripheral blood of subjects was quantitatively determined by liquid chromatography tandem-mass analysis (LC/MS/MS hereafter).

The specific LC/MS/MS system the inventors used for determination of the phosphatidylinositol (36:5) concentration in peripheral blood is described below.

After adding internal standard solution to the plasma from each subject, an organic solvent was added and deproteinization treatment was performed, the product was stirred and centrifuged, and the supernatant was recovered. The supernatant was dried by nitrogen gas and then reconstituted with organic solvent. The phosphatidylinositol (36:5) in blood was quantitatively determined by LC/MS/MS using the reconstituted solution as the determination solution. The Nexera (Shimadzu Corporation)-AP15000 (AB SCIEX) was used as the LC/MS/MS system. The blood components were separated and eluted under reverse phase conditions and then detection was performed by electrospray ionization in the negative ion mode and MRM (multiple reaction monitoring). The peak area of the phosphatidylinositol (36:5) and internal standard was analyzed by Analyst (AB SCIEX) and the area ratio was calculated. The phosphatidylinositol (36:5) in plasma was quantitatively determined using the value obtained by dividing the peak area ratio of each subject by the average value of the peak area ratio of the control group as the relative value.

Figure 3 summarizes the relative values of the amount of phosphatidylinositol (36:5) in plasma of the control group and the coronary artery disease group. The relative value of the phosphatidylinositol (36:5) in plasma of the control group was 100.

The phosphatidylinositol (36:5) concentration in the coronary artery disease group was approximately 79% lower than that of the control group, and a significant difference was observed (P < 0.0001) .

### Example 2

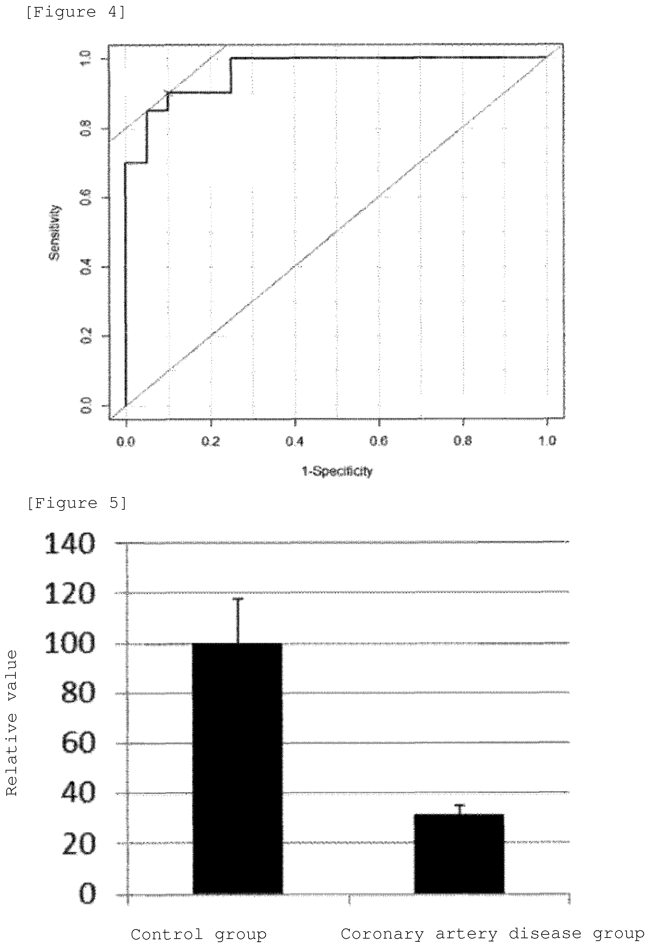

Figure 4 summarizes the results of ROC analysis of the results obtained in Example 1. The AUC was 0.963.

According to the results in Figure 4, the cut-off value when coronary artery disease is diagnosed using the relative value of the phosphatidylinositol (36:5) concentration is 43%, and a concentration that is the cut-off value or lower can be diagnosed as possible coronary artery disease.

### Example 3

### Diagnosis of coronary artery disease using lysophosphatidylinositol (20:5) in peripheral blood as molecular marker

In order to investigate the performance of lysophosphatidylinositol (20:5) as a novel marker for coronary artery disease, the lysophosphatidylinositol (20:5) concentration in plasma was compared by the same method as in Example 1 between 20 people in the control group and 20 people in the coronary artery disease group.

Figure 5 summarizes the relative values of the amount of lysophosphatidylinositol (20:5) in plasma of the control group and the coronary artery disease group. The relative value of the lysophosphatidylinositol (20:5) in the plasma of the control group was 100.

The phosphatidylinositol (20:5) concentration in the coronary artery disease group was approximately 69% lower than in the control group, and a significant difference was observed (P < 0.0001).

### Example 4

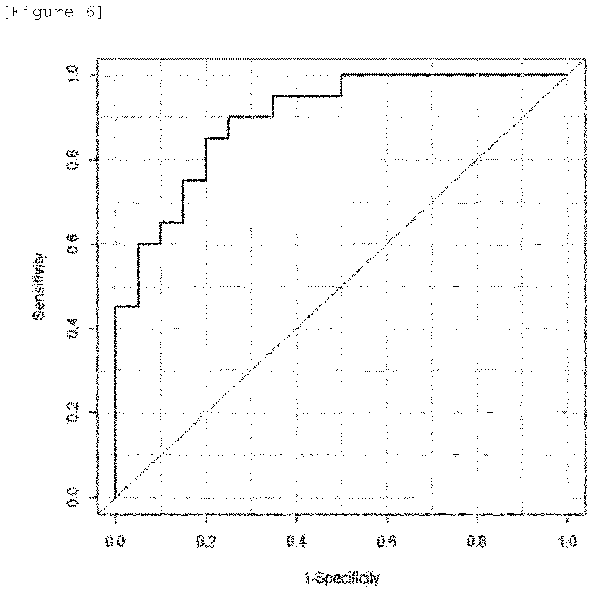

Figure 6 summarizes the results of ROC analysis of the results obtained in Example 3. The AUC was 0.897.

According to the results in Figure 6, the cut-off value when coronary artery disease is diagnosed using the relative value of the lysophosphatidylinositol (20:5) concentration is 44%, and a concentration that is the cut-off value or lower can be diagnosed as possible coronary artery disease.

The above-mentioned confirmed that phosphatidylinositol (36:5) or lysophosphatidylinositol (20:5) can be quantitatively determined in peripheral blood and can be used as a molecular marker for coronary artery disease.

## Claims

1. An *in vitro* method for detecting coronary artery disease, comprising a step for determining the amount of phosphatidylinositol (36:5) in a blood sample derived from a subject, wherein the fact that the determined value is smaller than a reference value corresponding to the amount of phosphatidylinositol (36:5) in a control sample derived from a person without coronary artery disease, is used as an indicator that the test sample is a sample derived from a person with coronary artery disease.

2. Use of phosphatidylinositol (36:5) as a marker for the *in vitro* detection of coronary artery disease.

## Patentansprüche

1. In-vitro-Verfahren zum Nachweis von koronarer Herzkrankheit, umfassend einen Schritt, bei dem man die Menge an Phosphatidylinositol (36:5) in einer von einem Subjekt stammenden Blutprobe bestimmt, wobei die Tatsache, dass der bestimmte Wert kleiner ist als ein Vergleichswert, der der Menge an Phosphatidylinositol (36:5) in einer von einer Person ohne koronarer Herzkrankheit stammenden Vergleichsprobe entspricht, als Indikator dafür genutzt wird, dass es sich bei der Testprobe um eine von einer Person mit koronarer Herzkrankheit stammende Probe handelt.

2. Verwendung von Phosphatidylinositol (36:5) als Marker zum In-vitro-Nachweis von koronarer Herzkrankheit.

## Revendications

1. Procédé *in vitro* pour la détection d'une maladie coronarienne, comprenant une étape pour la détermination de la quantité de phosphatidylinositol (36:5) dans un échantillon de sang issu d'un sujet, le fait que la valeur déterminée soit plus petite qu'une valeur de référence correspondant à la quantité de phosphatidylinositol (36:5) dans un échantillon de contrôle issu d'une personne sans maladie coronarienne, étant utilisé comme un indicateur que l'échantillon de test est un échantillon issu d'une personne comportant une maladie coronarienne.

2. Utilisation de phosphatidylinositol (36:5) en tant que marqueur pour la détection *in vitro* d'une maladie coronarienne.
